# EUROPEAN PATENT SPECIFICATION

(11) **EP 3 952 912 B1**
(45) Date of publication and mention of the grant of the patent: **23.04.2025**
(21) Application number: 20715106.9
(22) Date of filing: 06.04.2020
(51) Int. Cl.: A61K 47/18, A61K 31/337, A61K 31/4745, A61K 31/555, A61K 38/00

(54) **CELL-PENETRATING CONJUGATE SYSTEMS**
ZELLENPENETRIERENDE KONJUGATSYSTEME
SYSTÈMES DE CONJUGUÉS PÉNÉTRANT LES CELLULES

(30) Priority: 10.04.2019 EP 19168314
(43) Date of publication of application: 16.02.2022
(73) Proprietor: Danmarks Tekniske Universitet, 2800 Kongens Lyngby (DK)
(72) Inventor: SRIVASTAVA, Sarvesh, Kumar, 2800 Kgs. Lyngby (DK); VEIGA, Gael, Clergeaud, 1415 Copenhagen K (DK); BOISEN, Anja, 3460 Birkerød (DK)
(74) Representative: Plougmann Vingtoft a/s
(86) International application number: PCT/EP2020/059771
(87) International publication number: WO 2020/207974

(56) References cited:
- US-A1- 2004 220 264
- US-B2- 9 949 952
- BRIGITTE DESCHREVEL ET AL: "Thermodynamic parameters monitoring the equilibrium shift of enzyme-catalyzed hydrolysis/synthesis reactions in favor of synthesis in mixtures of water and organic solvent", BIOTECHNOLOGY AND BIOENGINEERING, vol. 81, no. 2, 25 November 2002 (2002-11-25), pages 167 - 177, XP055699900, ISSN: 0006-3592, DOI: 10.1002/bit.10458

## Description

### Technical field of the invention

The present invention relates to the in vitro use of a cell-penetrating conjugate system or a formulation incorporating the same in transporting e.g. therapeutically active agents, such as chemotherapeutics, anti-diabetic drugs and immunostimulators across the cellular membranes of mammalian cells, including human cells. The invention also relates to in vitro method of transporting a cargo molecule across a cellular membrane of mammalian cells. Furthermore, the invention also relates to a cell-penetrating conjugate system or a formulation incorporating the same for transporting a cargo molecule across a cellular membrane of mammalian cells.

### Background of the invention

The cellular membrane represents a significant barrier towards active uptake of extracellular molecules, and in turn, also hampers targeted delivery of therapeutic substances. A very limited number of molecules can passively be transported into a cell depending upon their molecular weight, polarity and net charge.

On the other hand, active transport is governed by receptor-mediated endocytosis or via ATP-binding cassette transporters. In addition, molecules may be artificially facilitated inside a cell by means of physical techniques like electroporation, sonication, and microinjection; or chemically transported by lipids, cell penetrating peptides, polymers, liposomes, viral/phage delivery among others. Clearly, most of these methods are very case specific with limited applications beyond in vitro models.

Cell Penetrating Peptides (CPPs) have previously been applied as delivery vectors for intracellular delivery of a variety of cargo molecules and delivery vehicles including imaging agents, small-molecule drugs, liposomes, and biopharmaceuticals including oligonucleotides, peptides and proteins.

US 9.949.952 B2 discloses use of helper esters or compositions comprising helper esters for the delivery of active ingredients through the skin, wherein the helper esters can be tryptophan esters, e.g. L-tryptophan pentyl ester, and wherein the active ingredient is e.g. melatonin, alpha-lipoic acid, vitamin E, vitamin D, glutathione, resveratrol, astaxanthin, beta carotene, vitamin A, vitamin C, vitamin B12, vitamin B6, folic acid, taurine. The helper esters can be used for treating skin disorders, gastroesophageal reflux disease, cancer, immune disorders, cardiovascular diseases, depression, seasonal affective disorder (SAD), circadian rhythm sleep disorders, insomnia, Alzheimer's disease, delirium, headaches, obesity, amyotrophic lateral sclerosis, tinnitus, irritable bowel syndrome, aging and autism spectrum disorders. *In vitro* penetration through human skin and transdermal delivery into the blood plasma is shown in US 9.949.952 B2.

WO18114863A1 relates to cell penetrating peptides (CPP's) with improved internalization properties and further relates to chimeric polypeptides which comprise such CPP linked to a peptide of interest. WO18114863A1 discloses that internalization of CPPs varies among peptides with different tryptophan content and that the tryptophan content can affect both CPP uptake mechanism and efficiency.

The scientific article, *"*Debmalya Bhunia et al., J. Am. Chem. Soc., 2018, 140 (5), pp 1697-1714, Spatial Position Regulates Power of Tryptophan: Discovery of a Major-Groove-Specific Nuclear-Localizing, Cell-Penetrating Tetrapeptide*",* discloses important role of tryptophan in cell penetration vehicles and shows how spatial positions of two tryptophans regulate the cellular entry and nuclear localization. The Debmalya Bhunia et al. article concludes that the disclosed short, non-toxic tetrapeptides have potential for cell penetration and nuclear localization.

The scientific article, *"*Li-Chun Hung et al., ACS Chem. Biol., 2017, 12 (2), pp 398-406, Heparin-Promoted Cellular Uptake of the Cell-Penetrating Glycosaminoglycan Binding Peptide, GBPECP, Depends on a Single Tryptophan*"* discloses that the intracellular penetration of Cell-Penetrating Glycosaminoglycan Binding Peptide depends on the presence of the tryptophan residue in its sequence compared with similar derivative peptides. The Li-Chun Hung et al. article concludes that Cell-Penetrating Glycosaminoglycan Binding Peptide shows substantial potential as a novel delivery therapeutic through rapid and effective internalization.

The scientific article, *"*Kristensen et al., Int. J. Mol. Sci. 2016, 17, 185; Applications and Challenges for Use of Cell-Penetrating Peptides as Delivery Vectors for Peptide and Protein Cargos*",* discloses that tryptophan residues improves the interaction with cell-surface-exposed glycosaminoglycans, which are believed to be involved in the process of endocytic CPP (cell penetrating peptide) uptake. The *Kristensen et al. article,* further discloses a direct positive correlation between the number of tryptophane residues in a basic CPP sequence and the binding affinity to GAGs in solution, with which they form stable aggregates and concludes that not only the presence, but also the specific positioning of tryptophane residues in a CPP sequence, influences the resulting efficiency in membrane permeation.

### Challenges in the prior art

- Internalization of therapeutic molecules inside the biological site of interest governs its therapeutic potential. Cellular membrane represents a significant physical barrier. To this end, several physical and chemical techniques have been reported, of which, cell penetrating peptides have garnered special attention. In particular, cells with low degree of cellular uptake, such as T-cells, internalization of molecules represents a considerable challenge. The inventors have demonstrated that it is possible with the claimed conjugate to allow real time tracking of T-cells owing to innate fluorescence of the claimed conjugate. Further, addition of a radiolabelled halide group (F, Cl, Br or I) to the conjugate will push it towards nuclear medicine imaging. This is a surprising and important discovery.
- CPPs are short amino acid sequences, which are able to enter cells; thus, they have been named "biological Trojan horses" and have been employed in cellular delivery of cargoes such as DNA, siRNA, Zr-labelled antibody for PET imaging among others.
- Despite significant progress with CPPs, the following challenges persists:
   a) The designing of amino acid sequences and its required tertiary structure is a significant challenge.
   b) Many CPPs require covalent conjugation with the molecule of interest, which greatly alters the inherent chemical stability and medical properties.
   c) For the ones, which facilitate non-covalent internalization, lack of sensitive methods of detection complicates design and mechanistic studies.

Further, while the role of tryptophan has long been investigated for multiple life science applications, limited information is available regarding its esterification products.

The cell-penetrating conjugate systems of the present invention possess at least the following advantages over the CPPs of the prior art:
i. Highly biocompatible: Made of naturally occurring amino acids with no observable cellular toxicity.
ii. Auto-fluorescent: Conjugate fluoresces with Ex/Em at 490/550nm allowing rapid quantification inside the cell.
iii. Uptake enhancer: Successfully internalizes molecules considered in the art as 'membrane impermeant' upon co-administration.
iv. Straight-forward synthesis: Utilizes one-pot click-like reaction for rapid synthesis of product in high quantities.
v. Simple purification: Upon synthesis, can be easily purified by a simple purification method

### Summary of the invention

A subject of the invention is the provision of a conjugate system or a formulation incorporating the same comprising a cell penetrating moiety, linked to a cargo molecule. The cargo molecule may be selected from the group consisting of a peptide, a protein, a nucleic acid, and a small molecule. In a particular embodiment, the cargo molecule is a therapeutic agent. Although not in accordance with the claimed invention, the conjugate system or a formulation incorporating the same may then be used as a medicament for use in treatment of cancer, diabetes and for use in immunotherapy.

The present invention relates to the in vitro use of a cell-penetrating conjugate system or a formulation incorporating the same in transporting e.g. therapeutically active agents, such as chemotherapeutics, anti-diabetic drugs and immunostimulators across the cellular membranes of mammalian cells, including human cells.

The invention also relates to in vitro method of transporting a cargo molecule across a cellular membrane of mammalian cells.

The structure of the conjugate system of the present invention holds the key: (i) an indole group facilitating hydrophobic interactions with the target molecule, i.e. the cargo molecule (generally hydrophobic) and (ii) a relatively hydrophilic tail which gets dissolved in water and penetrate into the cell. The inventors of the present invention postulate that this micellar, water-soluble structure facilitates drug delivery across the cellular membrane.

The charge on the aromatic structure of the conjugate system is important and must be capable of forming charged structures at physiological pH, much like a tryptophan molecule which is a zwitterion at physiological pH where the amino group is protonated (-NH₃⁺; pKₐ = 9.39) and the carboxylic acid is deprotonated ( -COO⁻; pKₐ = 2.38).

Calculated pKa value of 7.1 suggests that the conjugate system of the present invention is an ideal candidate for physiological conditions.

Thus, an object of the present invention relates to the in vitro use of a cell-penetrating conjugate system or a formulation incorporating the same for transporting a cargo molecule across a cellular membrane of mammalian cells;
wherein the cell-penetrating conjugate system is 4-hydroxybutyl L-tryptophanate represented by the following formula:

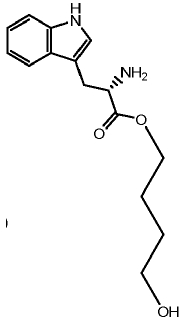

Another aspect of the present invention relates to an in vitro method of transporting a cargo molecule across a cellular membrane of mammalian cells;
wherein the method comprises a step of exposing the mammalian cells to the cargo molecule and conjugate system or a formulation incorporating the same;
wherein the cell-penetrating conjugate system is 4-hydroxybutyl L-tryptophanate represented by the following formula:

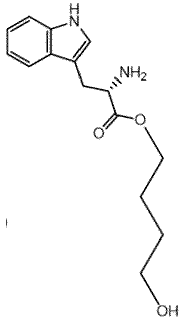

Still another aspect of the present invention relates to a cell-penetrating conjugate system or a formulation incorporating the same
for transporting a cargo molecule across a cellular membrane of mammalian cells;
wherein the cell-penetrating conjugate system is 4-hydroxybutyl L-tryptophanate represented by the following formula:

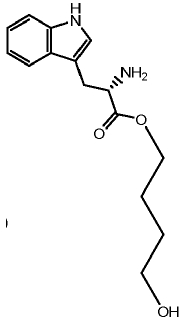

### Brief description of the figures

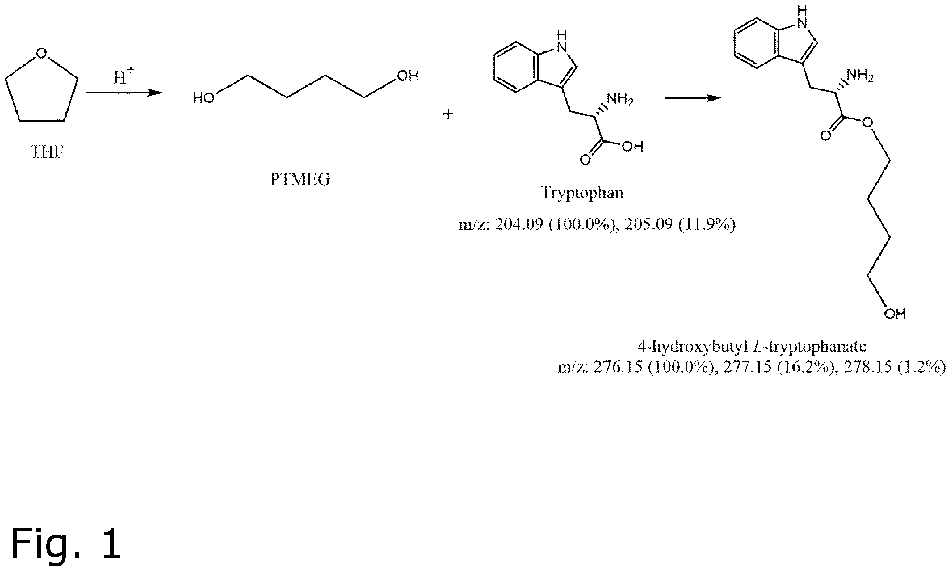

Figure 1 shows synthesis and m/z values of the cell-penetrating conjugate system, 4-hydroxybutyl L-tryptophanate.

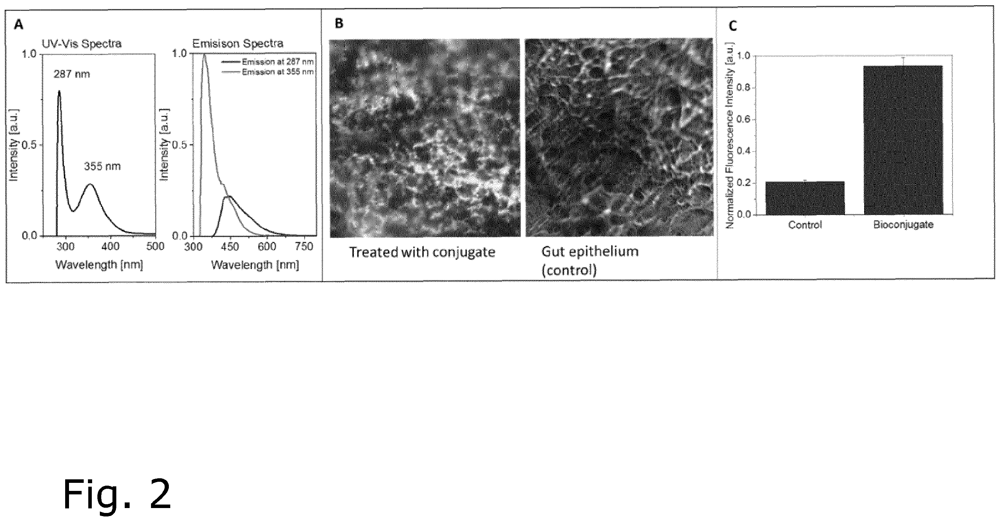

Figure 2 shows (A) UV-Vis properties of the conjugate; (B) Exposure of pig intestinal cross-section (with mucus) to the conjugate and (C) associated retention of fluorescence onto the treated tissue (see Example 2).

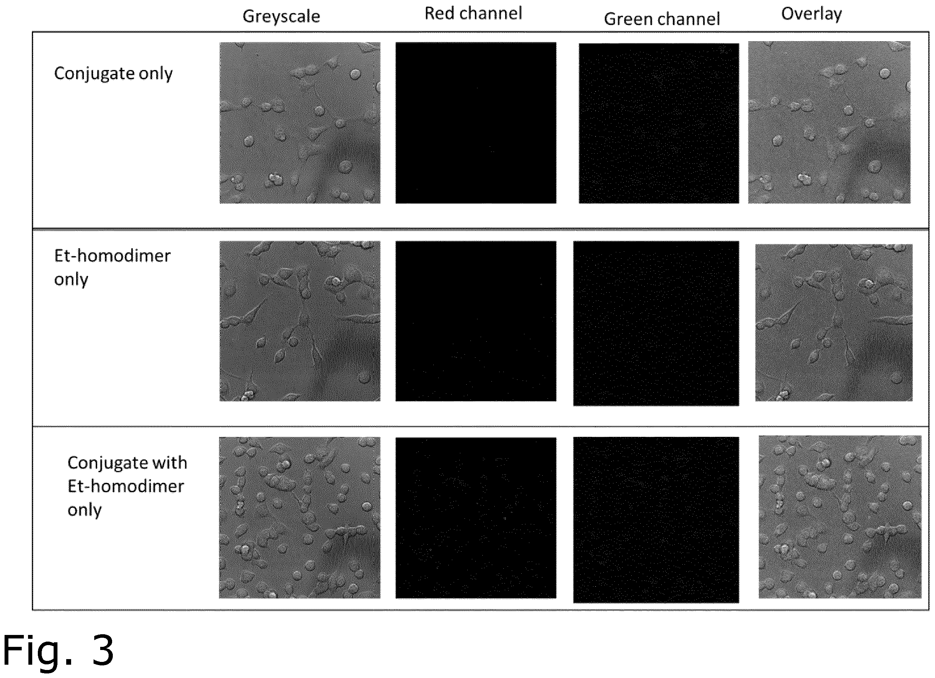

Figure 3 shows fluorescence confocal microscopy images of CT26 cells with Ethidium-homodimer (EtDi) (red) internalization with the conjugate (green). Images show that EtDi alone cannot penetrate into the cell, but when co-administered with the conjugate the EtDi got internalized into the cells.

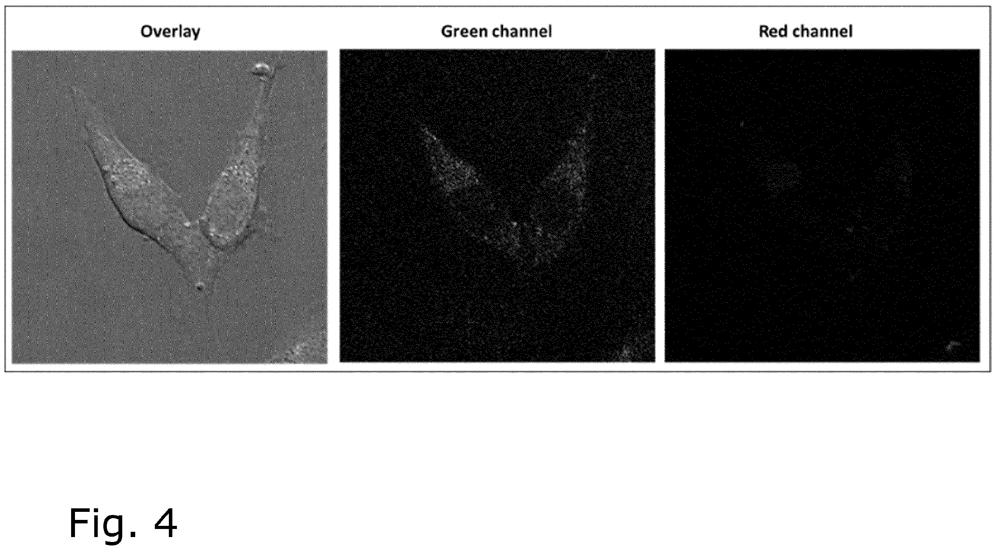

Figure 4 shows fluorescence confocal microscopy images of Ethidium-homodimer (EtDi) internalization at a single cellular level. The images show that the conjugate system (green) carrying the EtDi (red) can internalize into the cell.

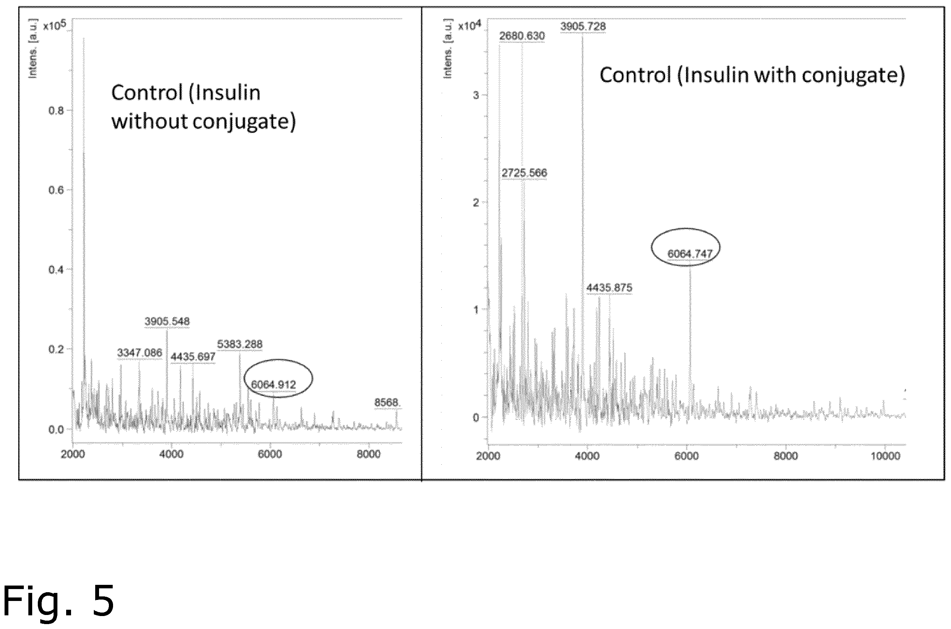

Figure 5 shows MALDI-TOF spectra of insulin (Novorapid ^{®}, insulin pen) internalization with the conjugate system (over 4-hours).

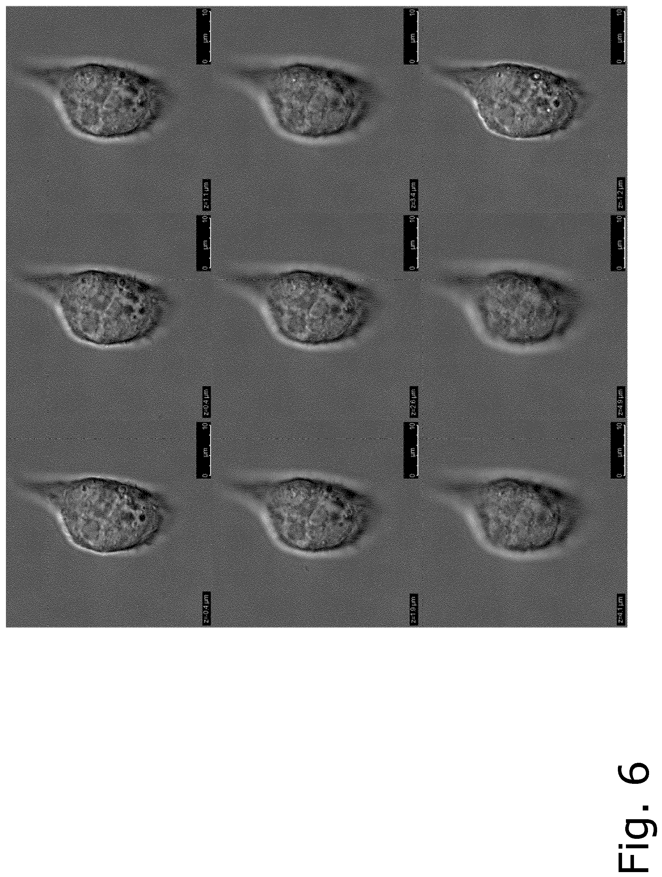

Figure 6 shows Z-stack confocal imaging which further confirmed internalization of the EtDi dye. Z-stack analysis of a single cell level showed internalization of both the EtDi and conjugate shows frames extracted from Z-stack showing internalized conjugate (green) with EtDi (red) across the cell (z-axis).

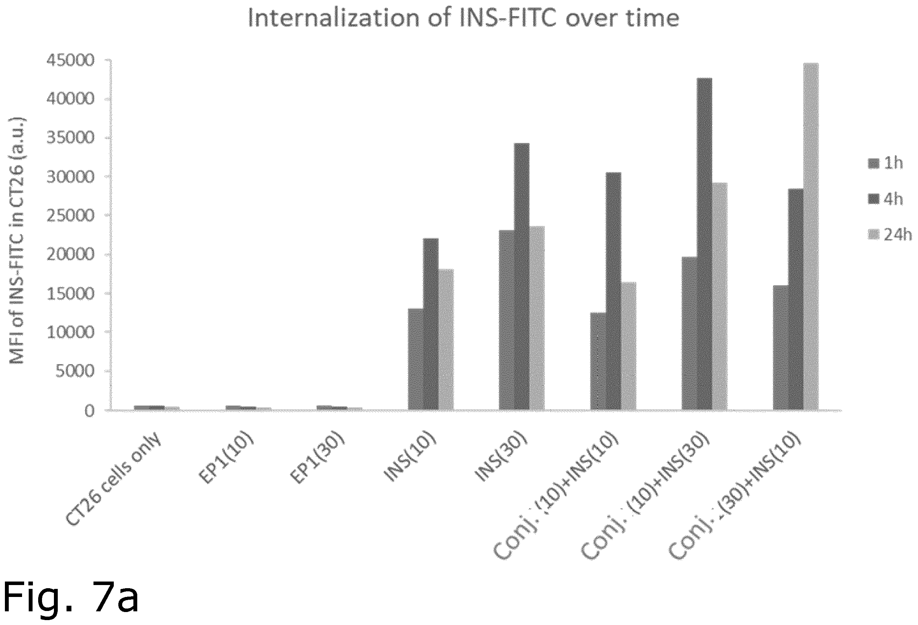

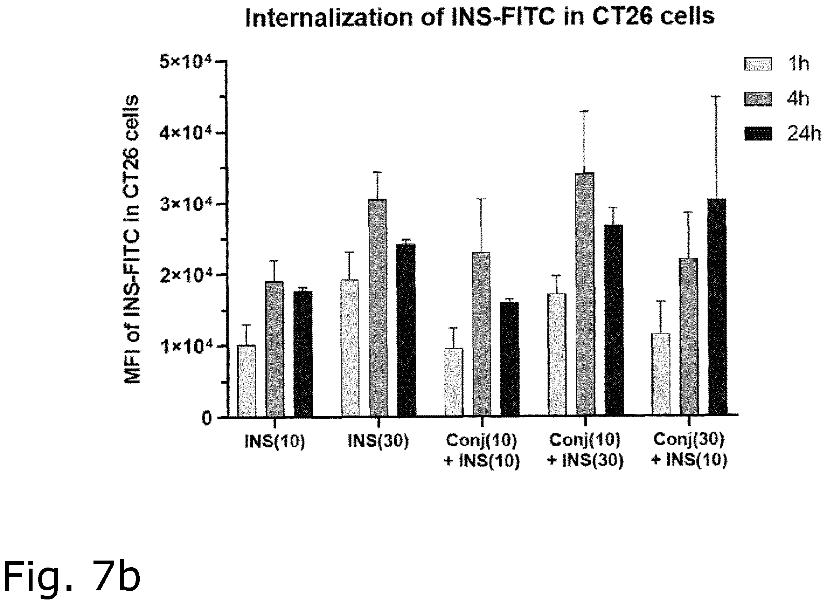

Figure 7 (7a-b) shows internalization of fluorescently labelled Insulin-FITC (INS-FITC) in CT-26 cells (in duplicate). The extent of INS-FITC internalization was measured by flow cytometry and mean fluorescent intensity (MFI) data clearly confirmed greater internalization when co-administered with the conjugate, hydroxybutyl L-tryptophanate. In the micromolar concentration ratio of 10:10, 4 hours seems to be the optimal time with a percentage increase of 20.5%. Interestingly, increasing the concentration of insulin to conjugate (30:10), showed an increase of 11.4% which remained constant from 4 to 24 hours. However, increasing the concentration of conjugate to insulin (30:10) yielded a percentage increase of around 72.3% after 24 hours of incubation compared to free insulin at the same concentration. This study clearly confirmed enhanced internalization of insulin.

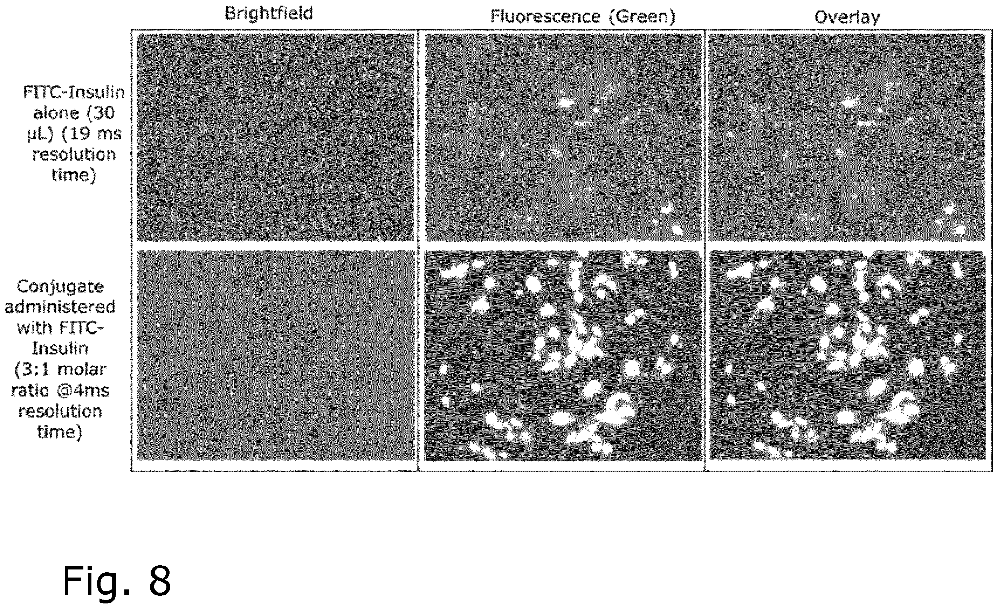

Figure 8 shows fluorescent imaging of CT-26 cells after 24 hours treatment with insulin-FITC with and without the conjugate 4-hydroxybutyl L-tryptophanate. The resolution time had to be significantly decreased in case of co-administration to avoid image saturation due to enhanced fluorescence (as a result of Ins-FITC internalization). No observable fluorescence was observed in case of 'conjugate alone' post 24-hours incubation.

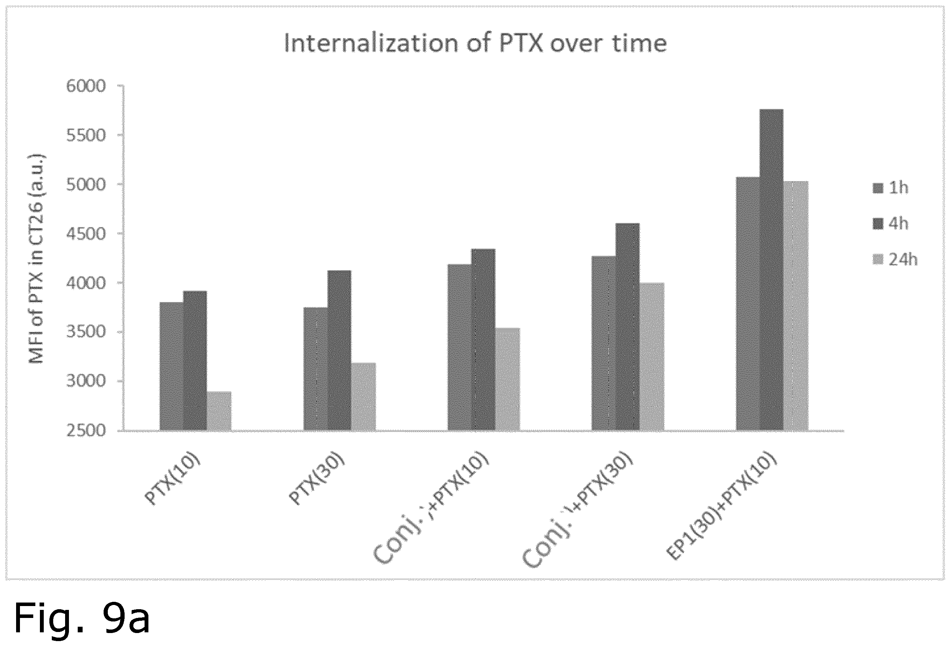

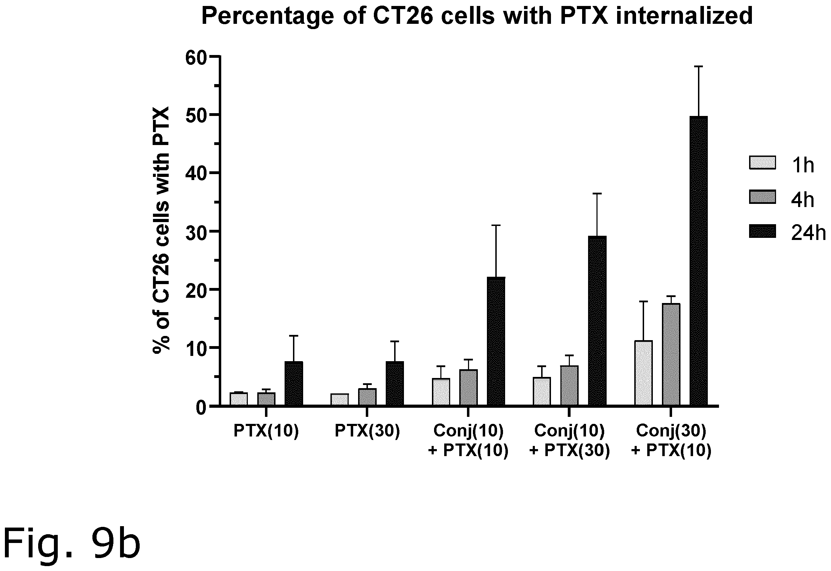

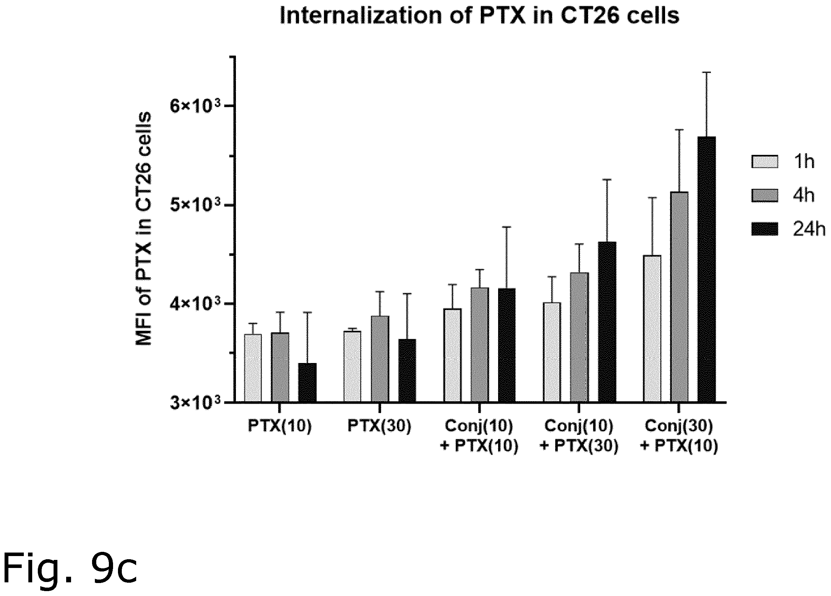

Figure 9 (9a-c) shows internalization of Paclitaxel (PTX) internalization in CT-26 cells measured by flow cytometry (duplicate). (b) shows the percentage of CT-26 cells that have internalized PTX. There is a remarkable increment in the percentage of cells containing PTX when co-administered with the conjugate, especially after 24 hours incubation. (c) depicts the amount of PTX internalization expressed as mean fluorescence intensity (MFI). Keeping the conjugate 4-hydroxybutyl L-tryptophanate concentration at 10 µM post-24 hours, the percentage increase in PTX internalization between treatments with or without the conjugate, was found to be 22.3% (ratio 10:10) and 27.1% (ratio 10:30). This indicates that the concentration of conjugate 4-hydroxybutyl L-tryptophanate and the drug-to-conjugate ratio influences the internalization of drug and was confirmed upon increasing the conjugate 4-hydroxybutyl L-tryptophanate concentration to 30 µM (ratio: 30:10) where percentage of PTX internalization was increased to 20.6% (1 hour), 32.4% (4 hours) and 56.2% (24 hours).

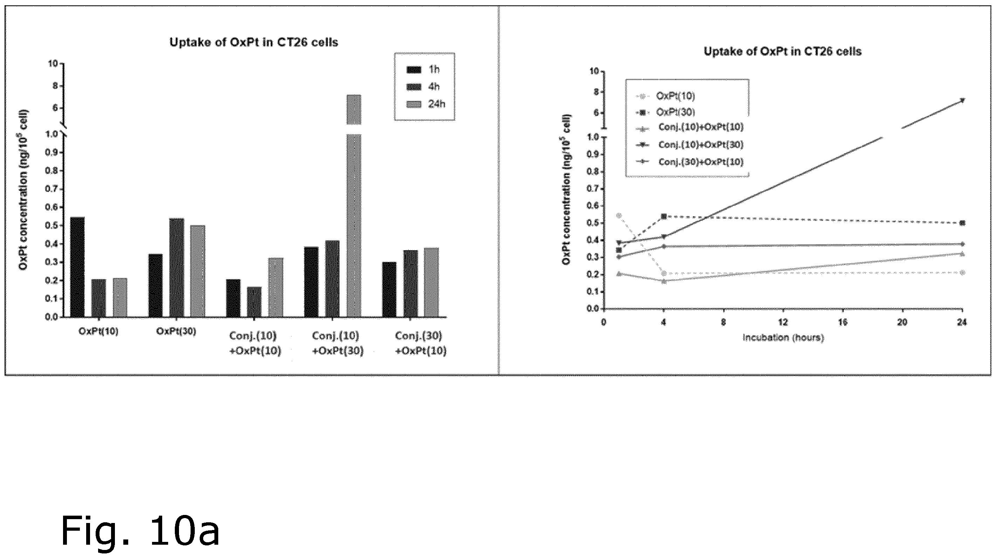

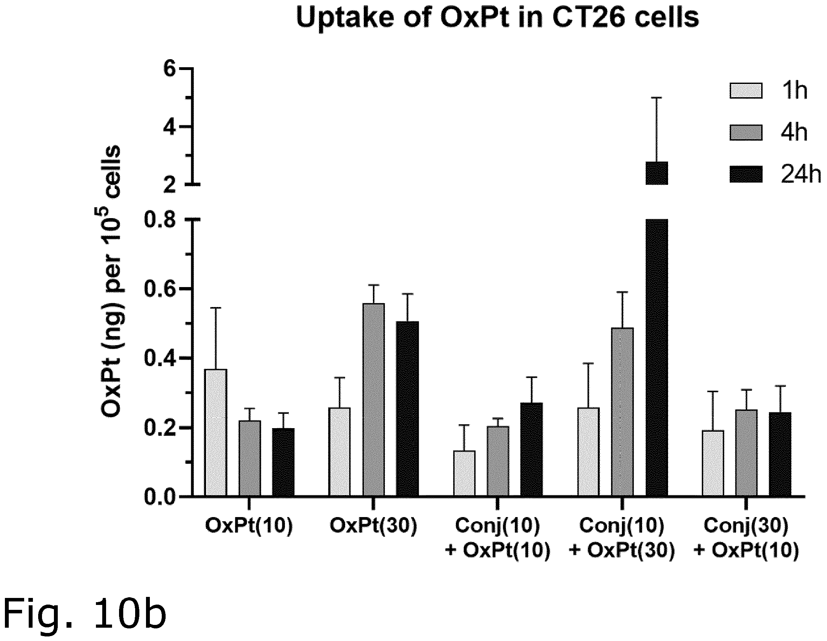

Figure 10 (10a-b) shows uptake of Oxaliplatin (OxPt) by CT-26 cells measured by ICP-MS (in triplicate). When cells were treated with 10µM OxPt the internalization was enhanced by 37.6% and 23.5% when co-administered with the conjugate 4-hydroxybutyl L-tryptophanate during 24 hours at 10:10 and 30:10 molar ratios, respectively. Remarkably, upon treating the cells with 30µM OxPt alone, there was no apparent increase on the OxPt uptake over time, peaking at 4h co-incubation. However, when co-administered with the conjugate at 10:30 ratio (Conj.:OxPt), the internalization percentage increased to 451%.

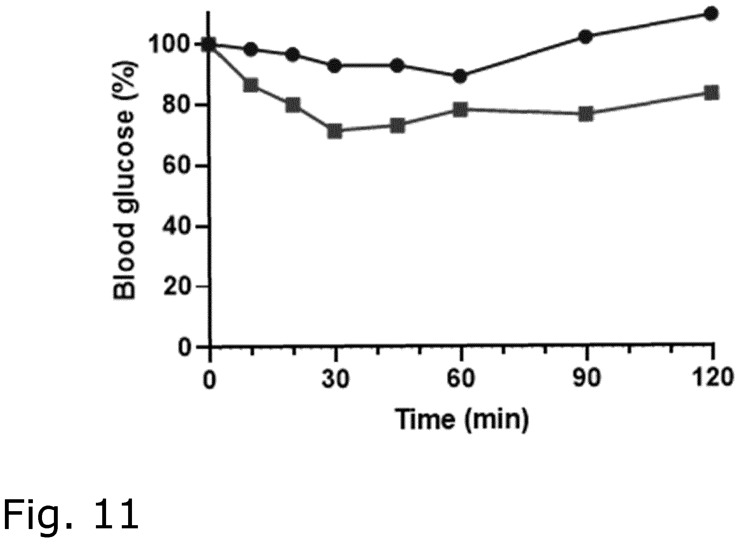

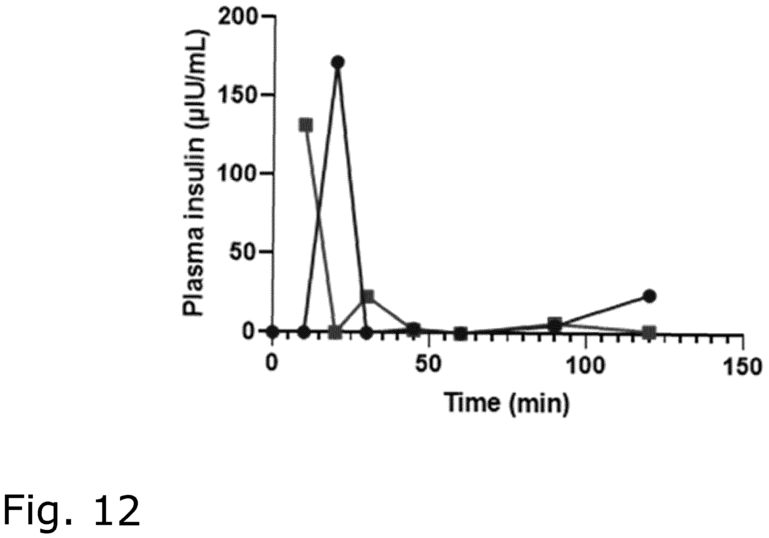

Figures 11-12 show the results of an in vivo insulin delivery and bioavailability experiment carried out on Sprague-Dawley rats (n=2) (rat 1 = •) (rat 2 = ■). 1.5 mg of insulin (sodium salt), together with the conjugate, 4-hydroxybutyl L-tryptophanate (0.5 mg) was loaded in an enteric-coated Eudragit^{®} L-100) rat capsule for oral gavaging in Sprague-Dawley rats. Insulin dosage was estimated to be ~120 I.U. The capsule was mechanically guided across the duodenum and rat-tail plasma samples were collected for a period of 3 hours (at specified periodic intervals). Blood glucose measurement was made with Glucometer and plasma insulin concentration via ELISA. Within the first hour of administration ~20% drop in blood sugar was observed (figure 11) along with increase in insulin plasma levels (~120µlU/mL; quantified via ELISA) (figure 12). Detection of insulin via ELISA, upon oral administration, suggests that the conjugate can be used to deliver biologics in therapeutic quantities.

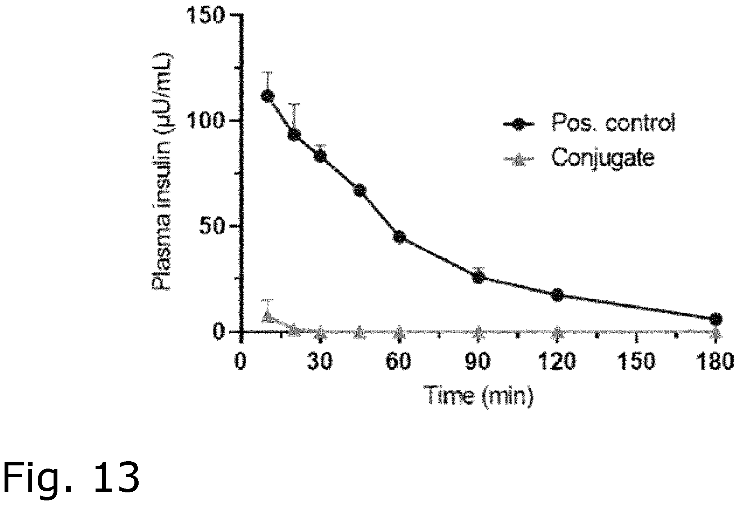

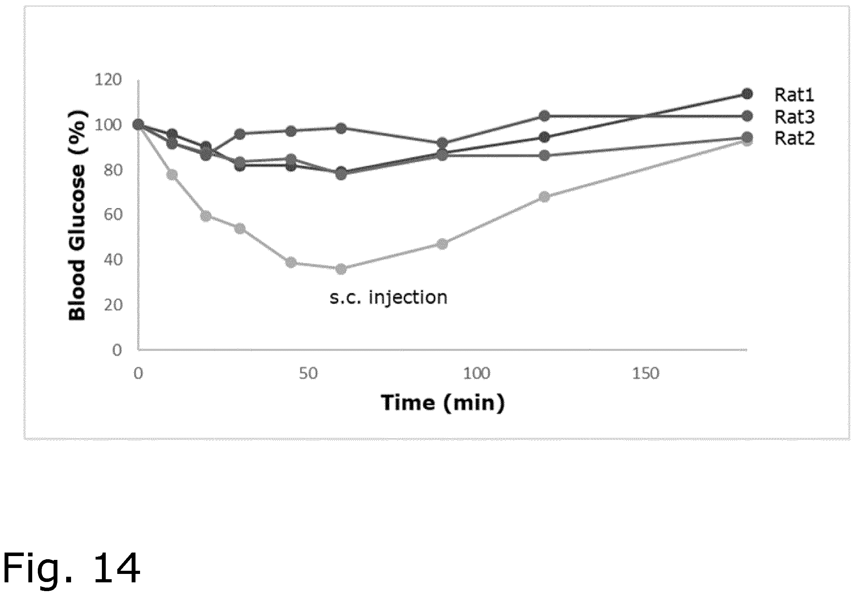

Figures 13 and 14 shows results of an in vivo insulin delivery and bioavailability experiment carried out on Sprague-Dawley rats (n=3). 1.1 mg of insulin (sodium salt; ~80 I.U ), together with the conjugate, 4-hydroxybutyl L-tryptophanate (8 mg) was loaded in an enteric-coated Eudragit^{®} L-100) rat capsule for oral gavaging in Sprague-Dawley rats. The capsule was mechanically guided across the duodenum and rat-tail plasma samples were collected for a period of 3 hours (at specified periodic intervals). Blood glucose measurement was made with Glucometer and plasma insulin concentration via ELISA. Within the twenty minutes of administration ~15% drop in blood sugar was observed (figure 14) along with increase in insulin plasma levels (~22µlU/mL; quantified via ELISA) (figure 13). Detection of insulin via ELISA, upon oral administration (1 out of 3 rats), suggests that the conjugate can be used to deliver biologics in therapeutic quantities. Specifically, this experiment was carried out as a separate in vivo study (n=3) (rats administered with insulin together with the conjugate, 4-hydroxybutyl L-tryptophanate = ▲) (positive control rats administered only with insulin = •), with the same protocol described in legends to figures 11-12, but with lower quantity of insulin (1.1 mg; ~80 µlU/mL quantified via ELISA) together with higher quantities of the conjugate (8 mg).

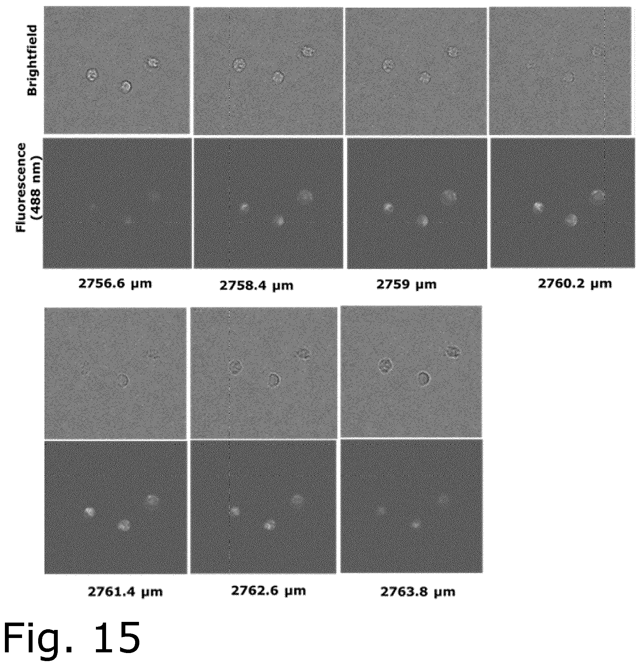

Figure 15 shows a fluorescent tagging in vitro experiment of T-cells. More specifically, figure 15 shows confocal fluorescence image (Z-stack) of mouse CD-4 (+) T-cells upon internalization (24 hours) with the conjugate, 4-hydroxybutyl L-tryptophanate. 100 µL 4-hydroxybutyl L-tryptophanate conjugate (1mg/ml) was added to the cell suspension solution (500 µL; 1 x 105 cells/mL) and incubated for 24 hours. Cells were washed with PBS buffer and analyzed via Z-stack imaging to confirm internalization of the 4-hydroxybutyl L-tryptophanate conjugate. The Z-stack imaging confirmed cellular internalization of the 4-hydroxybutyl L-tryptophanate conjugate resulting in characteristic fluorescence (488 nm) when viewed across the z-axis of the cell (~7 µm with a step of 1 µm each). Cell imaging was done for 0, 24, 48 and 72 hours - though 24 hours was found the optimum time frame.
Fluorescent tagging of T-cells supports that the 4-hydroxybutyl L-tryptophanate conjugate is efficient as a cellular internalization agent in immunological cells.

The present invention will now be described in more detail in the following.

### Detailed description of the invention

### IN VITRO USE

Although not in accordance with the claimed invention, the present disclosure relates to the in vitro use of a cell-penetrating conjugate system or a formulation incorporating the same for transporting a cargo molecule across a cellular membrane of mammalian cells, such as human cells;
wherein the cell-penetrating conjugate system comprises an amino acid ester of the general formula 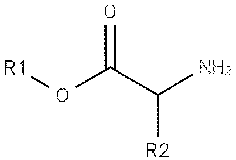 wherein;
R2 has a charge making it capable of forming charged structures at physiological pH, and
(a) when the conjugate system is tryptophane-based;

R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

An embodiment of the invention relates to the in vitro use of a cell-penetrating conjugate system or a formulation incorporating the same for transporting a cargo molecule across a cellular membrane of mammalian cells, such as human cells;
wherein the cell-penetrating conjugate system is 4-hydroxybutyl L-tryptophanate represented by the following formula:

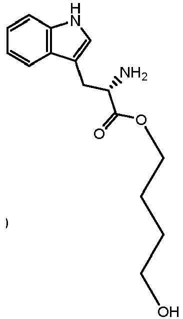

Although not in accordance with the claimed invention, the present disclosure relates to the in vitro use of a cell-penetrating conjugate system or a formulation incorporating the same having a molecular weight of at least 232 atomic mass unit (AMU) for transporting a cargo molecule across a cellular membrane of mammalian cells, such as human cells;
wherein the cell-penetrating conjugate system comprises an amino acid ester of the general formula 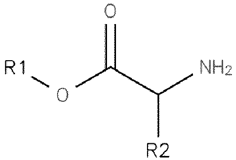 wherein;
R2 has a charge making it capable of forming charged structures at physiological pH, and
(a) when the conjugate system is tryptophane-based;

R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Another embodiment of the invention relates to the in vitro use of a cell-penetrating conjugate system or a formulation incorporating the same having a molecular weight of at least 232 atomic mass unit (AMU) for transporting a cargo molecule across a cellular membrane of mammalian cells, such as human cells;
wherein the cell-penetrating conjugate system is 4-hydroxybutyl L-tryptophanate represented by the following formula:

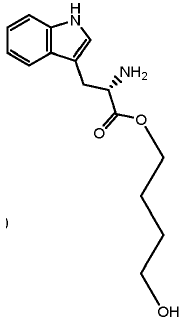

Although not in accordance with the claimed invention, the present disclosure relates to the in vitro use of a cell-penetrating conjugate system or a formulation incorporating the same for transporting a cargo molecule across a cellular membrane of mammalian cells, such as human cells;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system comprises an amino acid ester of the general formula 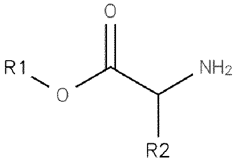 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Still another embodiment of the invention relates to the in vitro use of a cell-penetrating conjugate system or a formulation incorporating the same for transporting a cargo molecule across a cellular membrane of mammalian cells, such as human cells;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system is 4-hydroxybutyl L-tryptophanate represented by the following formula:

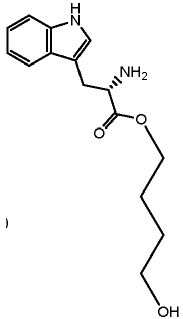

Although not in accordance with the claimed invention, the present disclosure relates to the in vitro use of a cell-penetrating conjugate system or a formulation incorporating the same, wherein the conjugate is having a molecular weight of at least 232 atomic mass unit (AMU) for transporting a cargo molecule across a cellular membrane of mammalian cells, such as human cells;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system comprises an amino acid ester of the general formula 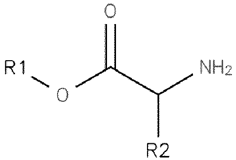 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Still another embodiment of the invention relates to the in vitro use of a cell-penetrating conjugate system or a formulation incorporating the same, wherein the conjugate is having a molecular weight of at least 232 atomic mass unit (AMU) for transporting a cargo molecule across a cellular membrane of mammalian cells, such as human cells;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system is 4-hydroxybutyl L-tryptophanate represented by the following formula:

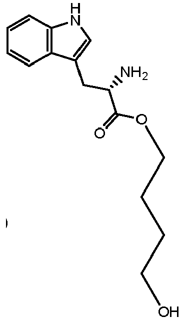

Another objective and advantage of using the cell-penetrating conjugate system or a formulation incorporating the same of the present invention is that it causes no negative effect on the viability of the penetrated cells and/or does not cause substantial membrane damage and/or leaves the penetrated cells healthy and intact.

### CONJUGATE SYSTEM FOR USE IN TREATMENT OF CANCER, DIABETES OR FOR USE IN IMMUNOTHERAPY

Although not in accordance with the claimed invention, the present disclosure relates to a cell-penetrating conjugate system or a formulation incorporating the same for transporting a cargo molecule across a cellular membrane of mammalian cells;
wherein the cell-penetrating conjugate system comprises an amino acid ester of the general formula 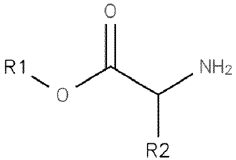 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester;
for use in treatment of cancer including ovarian cancer, breast cancer, pancreatic cancer and colon cancer, among others.

Although not in accordance with the claimed invention, the present disclosure relates to a cell-penetrating conjugate system or a formulation incorporating the same for use in treatment of cancer including ovarian cancer, breast cancer, pancreatic cancer and colon cancer, among others, wherein the conjugate system is 4-hydroxybutyl L-tryptophanate represented by the following formula:

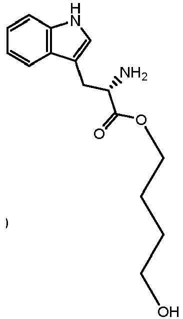

Although not in accordance with the claimed invention, the present disclosure relates to a cell-penetrating conjugate system or a formulation incorporating the same for transporting a cargo molecule across a cellular membrane of mammalian cells; wherein the cell-penetrating conjugate system comprises an amino acid ester of the general formula 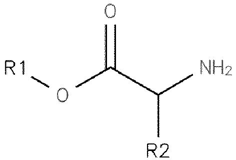 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester;
for use in treatment of diabetes.

Although not in accordance with the claimed invention, the present disclosure relates to a cell-penetrating conjugate system or a formulation incorporating the same for use in treatment of diabetes, wherein the conjugate system is 4-hydroxybutyl L-tryptophanate represented by the following formula:

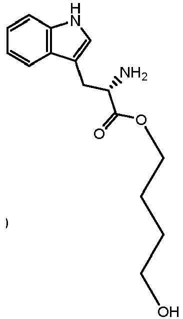

Although not in accordance with the claimed invention, the present disclosure relates to a cell-penetrating conjugate system or a formulation incorporating the same for transporting a cargo molecule across a cellular membrane of mammalian cells;
wherein the cell-penetrating conjugate system comprises an amino acid ester of the general formula 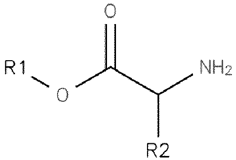 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester;
for use in immunotherapy.

Although not in accordance with the claimed invention, the present disclosure relates to a cell-penetrating conjugate system or a formulation incorporating the same for use in immunotherapy, wherein the conjugate system is 4-hydroxybutyl L-tryptophanate represented by the following formula:

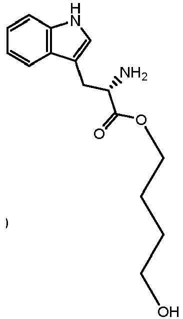

### METHOD OF TREATING CANCER AND DIABETES AND USES IN IMMUNOTHERAPY

Although not in accordance with the claimed invention, the present disclosure relates to a method of treating cancer including ovarian cancer, breast cancer, pancreatic cancer and colon cancer, among others, by using a cell-penetrating conjugate system or a formulation incorporating the same for transporting a cargo molecule across a cellular membrane of mammalian cells;
wherein the cell-penetrating conjugate system comprises an amino acid ester of the general formula 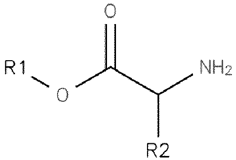 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to a method of treating cancer including ovarian cancer, breast cancer, pancreatic cancer and colon cancer, among others, by using a cell-penetrating conjugate system or a formulation incorporating the same wherein the conjugate system is 4-hydroxybutyl L-tryptophanate represented by the following formula:

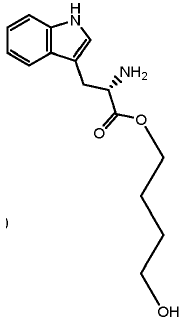

Although not in accordance with the claimed invention, the present disclosure relates to a method of treating diabetes by using a cell-penetrating conjugate system or a formulation incorporating the same for transporting a cargo molecule across a cellular membrane of mammalian cells; wherein the cell-penetrating conjugate system comprises an amino acid ester of the general formula 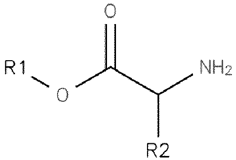 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to a method of treating diabetes by using a cell-penetrating conjugate system or a formulation incorporating the same wherein the conjugate system is 4-hydroxybutyl L-tryptophanate represented by the following formula:

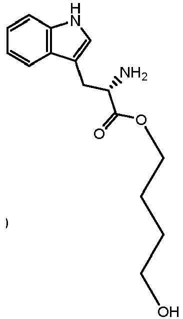

Although not in accordance with the claimed invention, the present disclosure relates to a method of immunotherapy using a cell-penetrating conjugate system or a formulation incorporating the same for transporting a cargo molecule across a cellular membrane of mammalian cells;
wherein the cell-penetrating conjugate system comprises an amino acid ester of the general formula 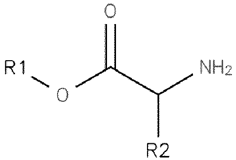 wherein;
R2 has a charge making it capable of forming charged structures at physiological pH, and (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to a method of immunotherapy using a cell-penetrating conjugate system or a formulation incorporating the same wherein the conjugate system is 4-hydroxybutyl L-tryptophanate represented by the following formula:

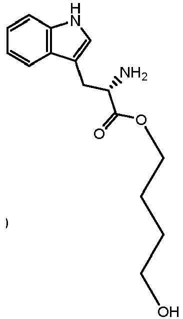

### IN VITRO METHOD

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the cell-penetrating conjugate system comprises an amino acid ester of the general formula 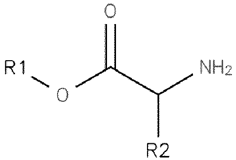 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

An embodiment of the invention relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the cell-penetrating conjugate system is 4-hydroxybutyl L-tryptophanate represented by the following formula:

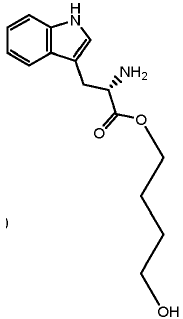

Although not in accordance with the claimed invention, the present disclosure relates to an in vitro method of transporting a cargo molecule across a cellular membrane of mammalian cells, such as human cells;
wherein the method comprises a step of exposing the mammalian cells to the cargo molecule and conjugate system or a formulation incorporating the same;
wherein the conjugate system is having a molecular weight of at least 232 atomic mass unit (AMU);
wherein the cell-penetrating conjugate system comprises an amino acid ester of the general formula 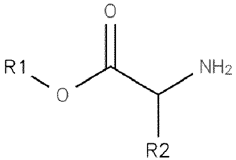 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the conjugate system is having a molecular weight of at least 232 atomic mass unit (AMU);
wherein the cell-penetrating conjugate system is 4-hydroxybutyl L-tryptophanate represented by the following formula:

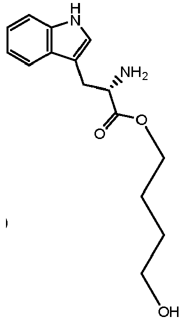

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system comprises an amino acid ester of the general formula 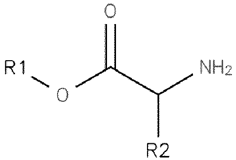 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system is 4-hydroxybutyl L-tryptophanate represented by the following formula:

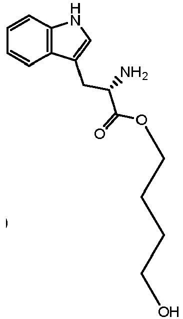

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system;
wherein the conjugate system is having a molecular weight of at least 232 atomic mass unit (AMU);
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system comprises an amino acid ester of the general formula 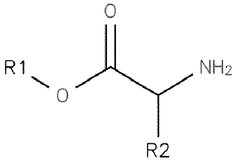 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the conjugate system is having a molecular weight of at least 232 atomic mass unit (AMU);
wherein the cargo molecule is a therapeutically active agent selected from the group consisting of a chemotherapeutic agent such as Camptothecin, Paclitaxel or Oxaliplatin, an anti-diabetic agent such as insulin, an immunosuppressant, an immunostimulators such as Resiquimod (R848) or a vaccine;
wherein the cell-penetrating conjugate system is 4-hydroxybutyl L-tryptophanate represented by the following formula:

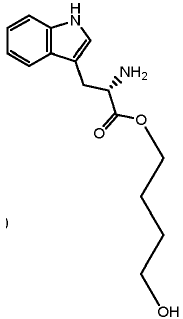

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the step of exposing the mammalian cells, such as human cells, to the cargo molecule and the conjugate system is by in vitro co-incubation for a period between 1-24 hours;
wherein the ratio between the cargo molecule and conjugate system is between 1:1 to 1:15;
wherein the cell-penetrating conjugate system comprises an amino acid ester of the general formula 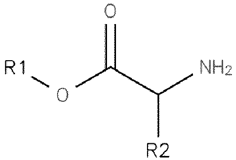 wherein;
   R2 has a charge making it capable of forming charged structures at physiological pH, and
   (a) when the conjugate system is tryptophane-based;
R1 is an alkyl functional group selected from the group consisting of methyl, ethyl, propyl, pentyl, hexyl, heptyl, octyl, nonyl, decyl, undecyl, dodecyl;
R2 is an indole conjugated with the amino acid ester.

Although not in accordance with the claimed invention, the present disclosure relates to an in vitro method of transporting a cargo molecule across a cellular membrane of mammalian cells, such as human cells;
wherein the method comprises a step of exposing the mammalian cells, such as human cells, to the cargo molecule and conjugate system or a formulation incorporating the same;
wherein the step of exposing the mammalian cells, such as human cells, to the cargo molecule and the conjugate system is by in vitro co-incubation for a period between 1-24 hours;
wherein the ratio between the cargo molecule and conjugate system is between 1:1 to 1:15;
wherein the cell-penetrating conjugate system is 4-hydroxybutyl L-tryptophanate represented by the following formula:

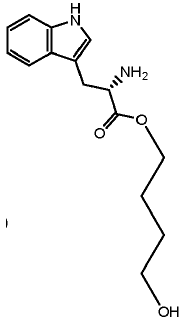

Another objective and advantage of using the cell-penetrating conjugate system or a formulation incorporating the same of the present invention is that it causes no negative effect on the viability of the penetrated cells and/or does not cause substantial membrane damage and/or leaves the penetrated cells healthy and intact.

### Definitions

Prior to discussing the present invention in further details, the following terms and conventions will first be defined:
Caco-2 cells: when used herein, Caco-2 cells (or Caco-2 cell lines) refers to a continuous line of heterogeneous human epithelial colorectal adenocarcinoma cells.

Cargo molecule (carrier molecule): when used herein, cargo molecule refers to e.g. the therapeutically active agent that is transported across the cell-membrane by the conjugate system of the present invention.

Cell-penetration: when used herein, "cell-penetrating" (or similar) refers to the transportation (ferrying) across the cellular membrane of e.g. the conjugate system when e.g. carrying a cargo molecule, thereby entering the inside of the cell (internalization). Cell-penetration is a seemingly energy-independent mechanism of cargo translocation across the cellular membrane that allows addressing of conjugated cargoes into the cytoplasm and nucleus. Cell-penetration is not receptor-dependent and thus, not cell-specific. Cell-penetration may provide a universal system for the delivery of cargo molecules into the cytoplasmic or nuclear compartments of any type of cell.

CT-26 cells: CT26 is an N-nitroso-N-methylurethane-(NNMU) induced, undifferentiated mouse colon carcinoma cell line.

Ethidium (EtDi) homodimer: generally considered in the art to be a membrane impermeant dye. This dye is a benchmark for live-dead staining, where the only way it can internalize into a cell (and stain it red), is if there is a membrane rupture.

Ferrying: when used herein, "ferrying" means transportation across the cell-membrane of e.g. a therapeutically active agent with the conjugate system of the present invention.

FICT: refers to fluorescein isothiocyanate.

"Indole conjugated with the amino acid ester": when used herein, "indole conjugated with the amino acid ester" refers to the fact that an indole group can undergo an electrophilic substitution, mainly at position 3 of C-atom (shown below).

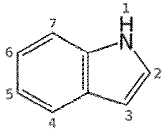

At this position, 4-hydroxybutyl(S)-2-aminobutanoate (the aliphatic portion of the conjugate of the invention) is attached. "Indole conjugated with the amino acid ester" may therefore be re-written as "Indole undergoing substitution at position 3 of C-atom with an n-hydroxyalkyl n-aminoalkanoate" or "indole ring undergoing a substitution at C-3 with an aminoalkanoate ester"

Internalization: when used herein, "internalization" (or similar) refers to the crossing of the cellular membrane and entering into the inside of the cell of e.g. the conjugate system, e.g. when carrying a cargo molecule.

m/z values: mass-to-charge ratio ("m" stands for mass and "z" stands for charge) Oxaliplatin (Ox): is an platinum-based anti-cancer drug (chemotherapeutic) used to treat colorectal cancer.

R1: when used herein, "R1" refers to an n-hydroxyalkyl, i.e. an alkyl functional group, where the alkyl part consists of methyl, ethyl, propyl, pentyl, hexyl, heptyl, octyl, nonyl, decyl, undecyl, dodecyl". Further, the chain-terminated hydroxyl (-OH) group can get oxidized into a carboxyl group (-C=O).

R2: when used herein, "R2" refers to an "Indole conjugated with the amino acid ester" where "indole conjugated with the amino acid ester" refers to the fact that an indole group can undergo an electrophilic substitution, mainly at position 3 of C-atom (shown below).

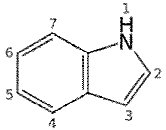

At this position, 4-hydroxybutyl(S)-2-aminobutanoate (the aliphatic portion of the conjugate of the invention) is attached. "Indole conjugated with the amino acid ester" may therefore be re-written as "Indole undergoing substitution at position 3 of C-atom with an n-hydroxyalkyl n-aminoalkanoate" or "indole ring undergoing a substitution at C-3 with an aminoalkanoate ester".

Resiquimod (R848): is an immunostimulatory drug that acts as an agonist of toll-like receptors (TLR) 7 and 8 to stimulate the innate immune system.

Tryptophane-based conjugate system: when used herein, and with reference to Figure 1, the term "tryptophane-based" conjugate system refers to a system where the core of the molecule is tryptophane (trp). For example, as can be seen from Figure 1, 4-hydroxybutyl L-tryptophanate is tryptophane-based (the same meaning applies *mutatis mutandis* for the term "phenylalanine-based", "tyrosine-based" and "histidine-based" conjugate systems).

Viability: when used herein, the term "without negatively affecting the viability of the penetrated cell" (or similar) refers a conjugate system capable of translocating into cells without causing substantial membrane damage, i.e. leaving healthy and fully intact membranes after being penetrated with the conjugate system of the present invention.

Z-stack imaging: when used herein, "Z-stack imaging" refers to a digital image processing technique which combines multiple images taken at different focus distances to give a resulting image with a greater depth of field than any of the individual source images

The invention will now be described in further details in the following non-limiting examples.

### Examples

### Example 1 Structure analysis of the conjugate system, 4-hydroxybutyl L-tryptophanate

Structure of the conjugate system, 4-hydroxybutyl L-tryptophanate has been predicted based upon reaction scheme and MALDI analysis (m/z) (figure 1).

### Example 2 Binding of conjugate system, 4-hydroxybutyl L-tryptophanate, to the pig intestine (gut epithelium) ex vivo

Biological functionality and compliance were tested utilizing intestinal tissue from pigs. A cross section of the pig intestine (1 cm²) was cleaned and subjected to the conjugate (1 mg/ml; 3.65 mM). After incubation (1 hour) of said tissue with the conjugate system, 4-hydroxybutyl L-tryptophanate, the tissue was washed and excess mucous layer removed by gentle physical exfoliation (scrapping via a spatula). It was observed that a clear fluorescence signal could be obtained, suggesting tissue binding and transmembrane penetration (see Figure 2a-c). Moreover, normalized fluorescence intensities on tissue were higher than controls incubated on a glass sample, suggesting that the intrinsic fluorescence can be enhanced via structural binding to living matter, such as intestinal tissue (gut epithelium).

The inherent green fluorescence of the conjugate system, 4-hydroxybutyl L-tryptophanate, was primarily characterized with a fluorospectrometer (Figure 2a).

The inventors observed the innate ability of the conjugate system, 4-hydroxybutyl L-tryptophanate, to deeply penetrate across mucosal layer on intestinal epithelium and bind to the tissue (gut epithelium) despite repeated washes was observed (Figure 2b).

In a controlled study, the inventors noticed that upon 1 hour of exposure the conjugate system, 4-hydroxybutyl L-tryptophanate, the pig intestine tissue attains its characteristic fluorescence as highlighted in Figure 2c.

As mentioned, the conjugate system, 4-hydroxybutyl L-tryptophanate, shows intrinsic fluorescence, which can be utilized in combination with other techniques to characterize molecule-target interactions.

Fluorescence based approaches, such as fluorescence microscopy and fluorescence binding assays rely on the integration of small molecular chemical probes (fluorophores) into a target molecule. However, fluorescence tagged molecules tend to interfere with targeted interactions, while hampering other analysis techniques due to their strong signal. Molecules with intrinsic fluorescent properties avoid these disadvantages and allow combinatorial sensing approaches. Intrinsic molecular fluorescence is dependent on the structural confirmation of the molecule.

Absorption and emission wavelengths determine the dynamic range of detection in a fluorescent molecule. Detection sensitivity is directly depended on fluorescence brightness, which is linked to the maximum absorption wavelength.

To further confirm if the conjugate system is binding onto the surface, or actually seeping inside the epithelium, cell study with internalization of Ethidium-homodimer was conducted.

### Example 3 Ethidium-homodimer internalization by using the conjugate system, 4-hydroxybutyl L-tryptophanate

Ethidium-homodimer was used as a model low-solubility drug candidate.

The inventors examined the cell penetrating effects (internalization) of the conjugate system, 4-hydroxybutyl L-tryptophanate, when transporting (ferrying) ethidium homodimer, generally considered in the art as a membrane impermeant dye and, thus, considered as a benchmark for live-dead staining, where the only way it can internalize into a cell is if there is a membrane rupture.

Surprisingly, by applying the conjugate system, 4-hydroxybutyl L-tryptophanate, internalization of 4-hydroxybutyl L-tryptophanate in living cells was clearly confirmed.

### Brief overview of experimental set-up:

CT-26 cells in culture were co-incubated with the conjugate system, 4-hydroxybutyl L-tryptophanate, -(1mg/ml) and Etidium-homodimer (2µl/ml) for 1 hour and observed under fluorescent confocal microscope. Working ratio was set as 1:3 and two reaction sets comprising of higher (80 µl) loading and standard loading (40 µl) were performed.
Emission bandwidth was set as 490-550nm and 590-680 nm

### Observation:

Internalization of Ethidium (EtDi)-homodimer (red fluorescence; 2 µl/ml) was confirmed within one hour of co-administration with the conjugate (1mg/ml) (see figure 3). The observed CT-26 cells were healthy with fully intact membranes.

The mean pixel intensity of confocal images showed an 8.67-fold increase of the EtDi fluorescence when the cells are treated with the EtDi combined with the conjugate compared with EtDi alone.

To further confirm that the dye is not merely sticking onto the cell surface, Z-stack imaging was performed which further confirmed internalization of the dye. Z-stack analysis of a single cell level showed internalization of both the EtDi and conjugate (see figure 6)

The above was the case for both higher loading (80µl) as well as the standard loading (40 µl) of the working mixture.

Cellular uptake of the conjugate system, 4-hydroxybutyl L-tryptophanate, as well as the cargo (carrier) molecule, ethidium homodimer, has been confirmed.

### Example 4 Efficiency of intracellular (transmembrane) delivery of Oxaliplatin-platinum (Pt)-based chemotherapeutic, with the conjugate system, 4-hydroxybutyl L-tryptophanate

### Brief overview of the experimental set-up:

### ICP-MS is a mass spectrometry technique for detecting extremely low concentrations of metals and non-metals in liquid samples. Pt-based chemotherapeutics are generally considered in the art to be highly-efficient in the mode of action but poor in their delivery (internalization).

CT26 cells were seeded in 24-well plate (100.000 cells / mL / well) exposed to Oxaliplatin (Ox-Pt, 10 µM and 30 µM) with/and without conjugate at the molar ratio of 1:1, 1:3 and 3:1 respectively. After 1, 4, 24 hours of incubation, media was discarded and the cells were washed with PBS twice, once with heparin (to remove anything associated externally with the cell membrane) and once more with PBS. Then the cells were detached from the wells using trypsin and cells were counted. Finally, each sample was diluted in HCl acid and further diluted in 2% HCl with (0.5ppb Ir), then Pt content was measured in the ICP-MS.

### Observation:

Overall, an increased amount of Pt signal was detected in the CT26 cells when the cells were treated with the combination of OxPt and the conjugate compared to the treatment with OxPt alone.

As can be seen from Figure 10 (10a-b), when cells were treated with 10µM OxPt the internalization was enhanced by 37.6% and 23.5% when co-administered with the conjugate 4-hydroxybutyl L-tryptophanate during 24 hours at 10:10 and 30:10 molar ratios, respectively. Remarkably, upon treating the cells with 30µM OxPt alone, there was no apparent increase on the OxPt uptake over time, peaking at 4h co-incubation. However, when co-administered with the conjugate at 10:30 ratio (Conj.:OxPt), the internalization percentage increased to 451%.

### Example 5 Cellular internalization of insulin (Novorapid^{®}) in CT-26 cells with 4-hydroxybutyl L-tryptophanate conjugate

### Brief overview of the experimental set-up:

MALDI-TOF is a standard technique for analysis of molecules based upon their mass/charge (m/z) ratio. In this study, the inventors exposed CT-26 cells with commercial insulin (20 µl) and conjugate system, 4-hydroxybutyl L-tryptophanate (20 µl). After 4 hours, the cells were sonicated (ruptured) and cell-debris removed; Resulting supernatant was subjected to MALDI analysis.

### Observation:

The inventors observed insulin peaks in both the samples as shown in figure 5. While the signal obtained in case of 'insulin with conjugate system, 4-hydroxybutyl L-tryptophanate is clearly distinct, indicating internalization of insulin into the CT-26 cells (MALDI TOF is not a quantitative technique).

Further, the inventors used insulin from a commercial insulin pen from Novo Nordisk A/S Denmark and the formulation must have some excipients which also enhances uptake. Therefore, this study will be repeated with the FITC-insulin (a fluorescent variant of insulin).

### Example 6 Internalization of FITC-insulin with 4-hydroxybutyl L-tryptophanate

As a follow-up study on the experiments of Example 5 (relating to insulin from a commercial insulin pen (from Novo Nordisk Denmark), i.e. an insulin formulation including excipients for enhancing cellular uptake), internalization experiments with FITC-insulin (a fluorescent variant of insulin) were carried out.

### Methodology:

CT26 colon cancer cells were seeded in 24-well plates at 100.000cells/well/mL. After 24 hours, cells were treated with INS-FITC at 10µM or 30µM concentration either alone or in combination with the conjugate 4-hydroxybutyl L-tryptophanate in three different molar ratios (1:1, 1:3 or 3:1). Treatments were co-incubated with the CT26 cells for 1h, 4h or 24h. Before observation, cells were washed several times with PBS, and with heparin, to ensure removal of compounds that might remain softly bound to cell membrane and not internalized. Then the washed cells were detached from the wells using trypsin, centrifuged and re-suspended in PBS for analysis by flow cytometry. In flow the cells were gated as singlets (single cell events) and then the fluorescence was recorded for each event in the specific FL channel where the compound has its emission max (INS-FITC: FL1 (525+/-40).

### Observation:

### Internalization of Insulin conjugated with FITC (INS-FITC)

While innate INS-FITC was at some extent internalized in all cells (i.e. 100% internalization in cells), mean fluorescent intensity (MFI) data clearly confirmed greater internalization when co-administered with the conjugate, hydroxybutyl L-tryptophanate. In the micromolar concentration ratio of 10:10, 4 hours seems to be the optimal time with a percentage increase of 20.5%. Interestingly, increasing the concentration of insulin to conjugate (30:10), showed an increase of 11.4% which remained constant from 4 to 24 hours. However, increasing the concentration of conjugate to insulin (30:10) yielded a percentage increase of around 72.3% after 24h incubation compared to free insulin at the same concentration. This study clearly confirmed enhanced internalization of insulin.

Post 24-hours, INS (30) and Conjugate (30)+Insulin (10) was also observed under a fluorescent microscope as shown in figure 8. Interestingly, the resolution time needs to be significantly decreased in case of co-administration to avoid image saturation due to enhanced fluorescence (as a result of Ins-FITC internalization). No observable fluorescence was observed in case of 'conjugate alone' post 24-hours incubation.

### Example 7 Time-dependent internalization of chemotherapeutic agent, Paclitaxel

Further experiments were carried out relating to a time-dependent internalization (transmembrane delivery) of the chemotherapeutic agent: Paclitaxel (a cyclodecane-based chemotherapeutic agent, a microtubule-inhibitor).

### Internalization of Paclitaxel (PTX)

PTX is a potent broad-spectrum chemotherapeutic to treat a number of cancers including ovarian cancer, breast cancer, pancreatic cancer and colon cancer, among others.

The results from the study shown in Figure 9b demonstrated a remarkable increment in the percentage of cells containing PTX when co-administered with the conjugate, especially after 24 hours incubation, where up to 50% of the CT-26 cells have internalized PTX when co-administered at a conjugate:PTX molar ratio of 30:10. Results are listed on table 1 below.

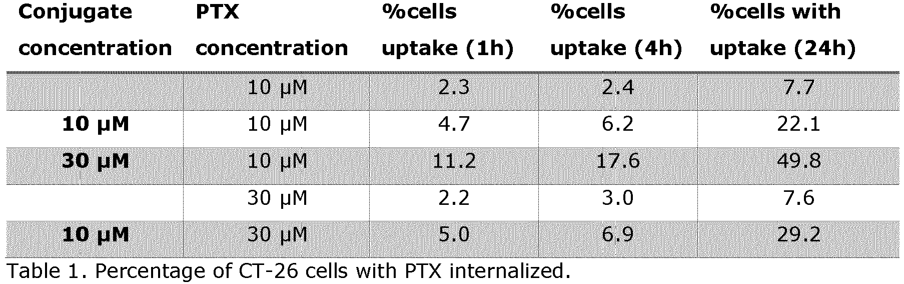

Figure 9c data depicts the amount of PTX internalization expressed as mean fluorescence intensity (MFI). Keeping the conjugate 4-hydroxybutyl L-tryptophanate concentration at 10 µM post-24 hours, the percentage increase in PTX internalization between treatments with or without the conjugate, was found to be 22.3% (ratio 10:10) and 27.1% (ratio 10:30). This indicates that the concentration of conjugate 4-hydroxybutyl L-tryptophanate and the drug-to-conjugate ratio influences the internalization of drug and was confirmed upon increasing the conjugate 4-hydroxybutyl L-tryptophanate concentration to 30 µM (ratio: 30:10) where percentage of PTX internalization was increased to 20.6% (1 hour), 32.4% (4 hours) and 56.2% (24 hours). See table 2 below.

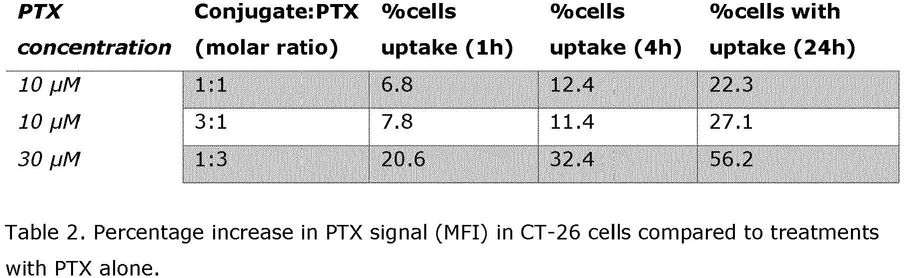

### Example 8 Fold increase internalization of the immuno-stimulator resiquimod (R848) in CT26 cells compared to treatments with R848 alone

### Protocol:

CT26 cells were seeded in 24-well plates (105 cells/well). After 24 hours the cells were treated with either 100 or 300µM concentration of resiquimod (R848) alone or in combination with the 4-hydroxybutyl L-tryptophanate conjugate. After 4 and 24 hours incubation cells were washed with cold PBS and with heparin solution (0.1 mg/mL heparin in PBS), and detached from the wells using trypsin. Then the cells were diluted in acetonitrile with 0.1% TFA and the amount of R848 was detected by HPLC.

### Key findings:

As can be seen from table 3 below, R848 (100 µM) combined with 4-hydroxybutyl L-tryptophanate conjugate (100 µM) increased to almost 5-times more the internalization of R848 compared to treating the cells for 4 hours with R848 (100 µM) alone. Treatment during 4 hours or 24 hours with combinations of R848 (100 µM) and 4-hydroxybutyl L-tryptophanate conjugate (300 µM) or R848 (300 µM) and 4-hydroxybutyl L-tryptophanate conjugate (100 µM) yielded between 1.1 to 1.8-times more cellular internalization of R848.

Overall the combination of R848 with 4-hydroxybutyl L-tryptophanate conjugate enhances its internalization of R848 in CT26 cells.

**Table 3: Fold increase internalization of resiquimod (R848) in CT26 cells compared to treatments with R848 alone**

| **Treatments** | **4 hours** | **24 hours** |
|---|---|---|
| 4-hydroxybutyl L-tryptophanate conjugate (100 µM)+ R848 (100 µM) | 4.9 | 0.9 |
| 4-hydroxybutyl L-tryptophanate conjugate (100 µM) + R848 (300 µM) | 1.4 | 1.8 |
| 4-hydroxybutyl L-tryptophanate conjugate (300 µM) + R848 (100 µM) | 1.5 | 1.1 |

### Example 9 In vivo insulin delivery and bioavailability experiment carried out on Sprague-Dawley rats

### Experimetal set-up:

1.5 mg of insulin (sodium salt), together with the conjugate, 4-hydroxybutyl L-tryptophanate (0.5 mg) was loaded in an enteric-coated Eudragit^{®} L-100) rat capsule for oral gavaging in Sprague-Dawley rats. Insulin dosage was estimated to be ~120 I.U. The capsule was mechanically guided across the duodenum and rat-tail plasma samples were collected for a period of 3 hours (at specified periodic intervals). Blood glucose measurement was made with Glucometer and plasma insulin concentration via ELISA.

### Observations:

Within the first hour of administration ~20% drop in blood sugar was observed (see figure 11) along with increase in insulin plasma levels (~120µlU/mL; quantified via ELISA) (see figure 12). Detection of insulin via ELISA, upon oral administration, suggests that the conjugate can be used to deliver biologics in therapeutic quantities.

Moreover, as shown in figure 13, an experiment was carried out as a separate in vivo study (n=3), with the same protocol described above, but with lower quantity of insulin (1.1 mg; ~80 µlU/mL quantified via ELISA) together with higher quantities of the 4-hydroxybutyl L-tryptophanate conjugate (8 mg) along with an s.c. control (1 I.U./kg).

### Example 10 Fluorescent tagging of T-cells

This fluorescent tagging in vitro experiment of T-cells shows confocal fluorescence image (Z-stack) of mouse CD-4 (+) T-cells cells upon internalization (24 hours) with the conjugate, 4-hydroxybutyl L-tryptophanate.

### Experimental set-up:

100 µL 4-hydroxybutyl L-tryptophanate conjugate (1mg/ml) was added to the cell suspension solution (500 µL; 1 x 105 cells/mL) and incubated for 24 hours. Cells were washed with PBS buffer and analyzed via Z-stack imaging to confirm internalization of the 4-hydroxybutyl L-tryptophanate conjugate. Cell imaging was done for 0, 24, 48 and 72 hours - though 24 hours was found the optimum time frame.

### Observations:

The Z-stack imaging confirmed cellular internalization of the 4-hydroxybutyl L-tryptophanate conjugate resulting in characteristic fluorescence (488 nm) when viewed across the z-axis of the cell (~7 µm with a step of 1 µm each). The fluorescent tagging of T-cells supports that the 4-hydroxybutyl L-tryptophanate conjugate is efficient as a cellular internalization agent in immunological cells.

## Claims

1. In vitro use of a cell-penetrating conjugate system or a formulation incorporating the same for transporting a cargo molecule across a cellular membrane of mammalian cells; wherein the conjugate system is 4-hydroxybutyl L-tryptophanate represented by the following formula:

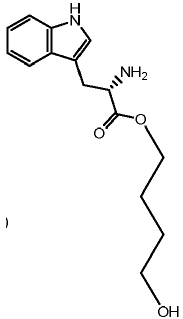

2. The in vitro use of a cell-penetrating conjugate system or a formulation incorporating the same according to claim 1, wherein the cell-penetrating conjugate system is having a molecular weight of at least 232 atomic mass unit (AMU).

3. The in vitro use of a cell-penetrating conjugate system or a formulation incorporating the same according to any of claims 1-2, wherein the cargo molecule is a therapeutically active agent.

4. The in vitro use of a cell-penetrating conjugate system or a formulation incorporating the same according to claim 3, wherein the therapeutically active agent is selected from the group consisting of a chemotherapeutic agent, an anti-diabetic agent, an immunosuppressant, an immunostimulator or a vaccine.

5. The in vitro use of a cell-penetrating conjugate system or a formulation incorporating the same according to claim 4, wherein the chemotherapeutic agent is Camptothecin, Paclitaxel or Oxaliplatin.

6. The in vitro use of a cell-penetrating conjugate system or a formulation incorporating the same according to claim 4, wherein the anti-diabetic agent is insulin.

7. The in vitro use of a cell-penetrating conjugate system or a formulation incorporating the same according to claim 4, wherein the immunostimulator is Resiquimod (R848).

8. The in vitro use of a cell-penetrating conjugate system or a formulation incorporating the same according to any of claims 1-2, wherein the cargo molecule is a labelling agent or a dye.

9. The in vitro use of a cell-penetrating conjugate system or a formulation incorporating the same according to claim 8, wherein the dye is an ethidium homodimer.

10. The in vitro use of a cell-penetrating conjugate system or a formulation incorporating the same according to any of claims 1-9, wherein the cells to be penetrated are selected from the group consisting of intestinal cells, cancer cells and immunological cells, such as T-cells.

11. The in vitro use of a cell-penetrating conjugate system or a formulation incorporating the same according to any of claims 1-10, wherein the mammalian cells are human cells

12. In vitro method of transporting a cargo molecule across a cellular membrane of mammalian cells;
wherein the method comprises a step of exposing the mammalian cells to the cargo molecule and conjugate system or a formulation incorporating the same;
wherein the cell-penetrating conjugate system is 4-hydroxybutyl L-tryptophanate represented by the following formula:

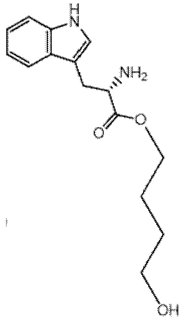

13. A cell-penetrating conjugate system or a formulation incorporating the same for transporting a cargo molecule across a cellular membrane of mammalian cells; wherein the cell-penetrating conjugate system is 4-hydroxybutyl L-tryptophanate represented by the following formula:

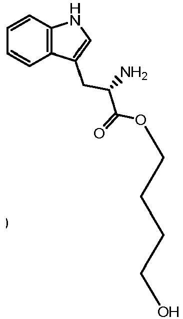

## Patentansprüche

1. In-vitro-Verwendung eines zellenpenetrierenden Konjugatsystems oder einer Formulierung, die dieses beinhaltet, zum Transportieren eines Cargo-Moleküls durch eine Zellmembran von Säugetierzellen; wobei das Konjugatsystem 4-Hydroxybutyl-L-tryptophanat ist, das durch die folgende Formel dargestellt wird:

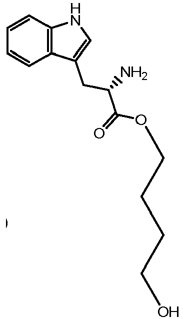

2. In-vitro-Verwendung eines zellenpenetrierenden Konjugatsystems oder einer Formulierung, die dieses beinhaltet, nach Anspruch 1, wobei das zellenpenetrierende Konjugatsystem ein Molekulargewicht von mindestens 232 atomaren Masseneinheiten (AMU) aufweist.

3. In-vitro-Verwendung eines zellenpenetrierenden Konjugatsystems oder einer Formulierung, die dieses beinhaltet, nach einem der Ansprüche 1-2, wobei das Cargo-Molekül ein therapeutischer Wirkstoff ist.

4. In-vitro-Verwendung eines zellenpenetrierenden Konjugatsystems oder einer Formulierung, die dieses beinhaltet, nach Anspruch 3, wobei der therapeutische Wirkstoff aus der Gruppe ausgewählt ist, die aus einem Chemotherapeutikum, einem Antidiabetikum, einem Immunsuppressivum, einem Immunstimulator oder einem Impfstoff besteht.

5. In-vitro-Verwendung eines zellenpenetrierenden Konjugatsystems oder einer Formulierung, die dieses beinhaltet, nach Anspruch 4, wobei das Chemotherapeutikum Camptothecin, Paclitaxel oder Oxaliplatin ist.

6. In-vitro-Verwendung eines zellenpenetrierenden Konjugatsystems oder einer Formulierung, die dieses beinhaltet, nach Anspruch 4, wobei das Antidiabetikum Insulin ist.

7. In-vitro-Verwendung eines zellenpenetrierenden Konjugatsystems oder einer Formulierung, die dieses beinhaltet, nach Anspruch 4, wobei der Immunstimulator Resiquimod (R848) ist.

8. In-vitro-Verwendung eines zellenpenetrierenden Konjugatsystems oder einer Formulierung, die dieses beinhaltet, nach einem der Ansprüche 1-2, wobei das Cargo-Molekül ein Markierungsmittel oder ein Farbstoff ist.

9. In-vitro-Verwendung eines zellenpenetrierenden Konjugatsystems oder einer Formulierung, die dieses beinhaltet, nach Anspruch 8, wobei der Farbstoff ein Ethidium-Homodimer ist.

10. In-vitro-Verwendung eines zellenpenetrierenden Konjugatsystems oder einer Formulierung, die dieses beinhaltet, nach einem der Ansprüche 1-9, wobei die zu durchdringenden Zellen aus der Gruppe ausgewählt sind, die aus Darmzellen, Krebszellen und immunologischen Zellen, wie T-Zellen, besteht.

11. In-vitro-Verwendung eines zellenpenetrierenden Konjugatsystems oder einer Formulierung, die dieses beinhaltet, nach einem der Ansprüche 1-10, wobei die Säugetierzellen menschliche Zellen sind

12. In-vitro-Verfahren für den Transport eines Cargo-Moleküls durch eine Zellmembran von Säugetierzellen;
wobei das Verfahren einen Schritt umfasst, bei dem die Säugetierzellen dem Cargo-Molekül und dem Konjugatsystem oder einer Formulierung, die dieses beinhaltet, ausgesetzt werden;
wobei das zellenpenetrierende Konjugatsystem 4-Hydroxybutyl-L-tryptophanat ist, das durch die folgende Formel dargestellt wird:

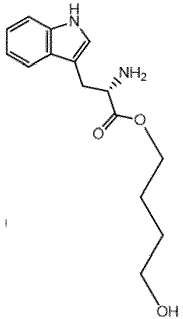

13. Zellenpenetrierendes Konjugatsystem oder dieses beinhaltende Formulierung für den Transport eines Cargo-Moleküls durch eine Zellmembran von Säugetierzellen;
wobei das zellenpenetrierende Konjugatsystem 4-Hydroxybutyl-L-tryptophanat ist, das durch die folgende Formel dargestellt wird:

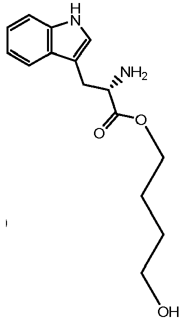

## Revendications

1. Utilisation in vitro d'un système conjugué de pénétration cellulaire ou d'une formulation incorporant celui-ci pour transporter une molécule cargo à travers une membrane cellulaire de cellules de mammifères ; dans laquelle le système conjugué est le 4-hydroxybutyle L-tryptophanate représenté par la formule suivante :

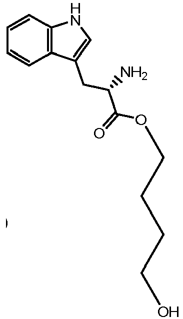

2. Utilisation in vitro d'un système conjugué de pénétration cellulaire ou d'une formulation incorporant celui-ci selon la revendication 1, dans laquelle le système conjugué de pénétration cellulaire a un poids moléculaire d'au moins 232 unités de masse atomique (AMU).

3. Utilisation in vitro d'un système conjugué de pénétration cellulaire ou d'une formulation incorporant celui-ci selon l'une quelconque des revendications 1 à 2, dans laquelle la molécule cargo est un agent thérapeutiquement actif.

4. Utilisation in vitro d'un système conjugué de pénétration cellulaire ou d'une formulation incorporant celui-ci selon la revendication 3, dans laquelle l'agent thérapeutiquement actif est sélectionné parmi le groupe constitué d'un agent chimiothérapeutique, d'un agent antidiabétique, d'un immunosuppresseur, d'un immunostimulateur ou d'un vaccin.

5. Utilisation in vitro d'un système conjugué de pénétration cellulaire ou d'une formulation incorporant celui-ci selon la revendication 4, dans laquelle l'agent chimiothérapeutique est la camptothécine, le paclitaxel ou l'oxaliplatine.

6. Utilisation in vitro d'un système conjugué de pénétration cellulaire ou d'une formulation incorporant celui-ci selon la revendication 4, dans laquelle l'agent antidiabétique est l'insuline.

7. Utilisation in vitro d'un système conjugué de pénétration cellulaire ou d'une formulation incorporant celui-ci selon la revendication 4, dans laquelle l'immunostimulateur est le Résiquimod (R848).

8. Utilisation in vitro d'un système conjugué de pénétration cellulaire ou d'une formulation incorporant celui-ci selon l'une quelconque des revendications 1 à 2, dans laquelle la molécule cargo est un agent de marquage ou un colorant.

9. Utilisation in vitro d'un système conjugué de pénétration cellulaire ou d'une formulation incorporant celui-ci selon la revendication 8, dans laquelle le colorant est un homodimère d'éthidium.

10. Utilisation in vitro d'un système conjugué de pénétration cellulaire ou d'une formulation incorporant celui-ci selon l'une quelconque des revendications 1 à 9, dans laquelle les cellules à pénétrer sont sélectionnées parmi le groupe constitué de cellules intestinales, de cellules cancéreuses et de cellules immunologiques, telles que les lymphocytes T.

11. Utilisation in vitro d'un système conjugué de pénétration cellulaire ou d'une formulation incorporant celui-ci selon l'une quelconque des revendications 1 à 10, dans laquelle les cellules de mammifères sont des cellules humaines

12. Procédé in vitro de transport d'une molécule cargo à travers une membrane cellulaire de cellules de mammifères ;
dans lequel le procédé comprend une étape d'exposition des cellules de mammifères à la molécule cargo et au système conjugué ou une formulation incorporant celui-ci ;
dans lequel le système conjugué de pénétration cellulaire est le 4-hydroxybutyle L-tryptophanate représenté par la formule suivante :

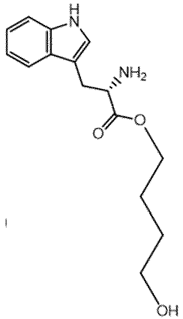

13. Système conjugué de pénétration cellulaire ou formulation incorporant celui-ci pour transporter une molécule cargo à travers une membrane cellulaire de cellules de mammifères ;
dans lequel le système conjugué de pénétration cellulaire est le 4-hydroxybutyle L-tryptophanate représenté par la formule suivante :
